Europäisches Patentamt

European Patent Office

Office européen des brevets

(11) Publication number: **0 262 805**
A1

# (12) EUROPEAN PATENT APPLICATION

(21) Application number: **87307715.0**

(22) Date of filing: **01.09.87**

(51) Int. Cl.4: **C12N 15/00** , C12C 11/00 ,
//C12N9/86

(30) Priority: **01.09.86 GB 8621118**

(43) Date of publication of application:
**06.04.88 Bulletin 88/14**

(84) Designated Contracting States:
**ES GR**

(71) Applicant: WHITBREAD & COMPANY PLC
Brewery Chiswell Street
London EC1Y 4SD(GB)

(72) Inventor: Lancashire, William Edward
21 Beaumont Green
Groby Leicestershire, LE6 0EP(GB)

(74) Representative: Perry, Robert Edward et al
GILL JENNINGS & EVERY 53-64 Chancery
Lane
London WC2A 1HN(GB)

(54) Stabilised polypeptides and their expression and use in fermentation.

(57) A yeast fermentation process, e.g. in brewing or distillation, in which the yeast DNA has been modified such that a selected polypeptide not naturally secreted by the yeast in a glycosylated form is expressed and secreted in the form of a glycosylated analogue.

EP 0 262 805 A1

## STABILISED POLYPEPTIDES AND THEIR EXPRESSION AND USE IN FERMENTATION

### FIELD OF THE INVENTION

The present invention relates to polypeptides which have been modified to infer increased stability, and to the genetic modification of yeasts which then both express and secrete such polypeptides in a brewing or other fermentation process.

### BACKGROUND OF THE INVENTION

Yeasts secrete polypeptides, and can be modified to secrete foreign polypeptides owing to the presence of a genetic secretion signal, e.g. the prepro-region of the "α-factor" sequence. Secretion may be accompanied by glycosylation if the polypeptide includes the "trigger" sequence Asn-X-Ser or Asn-X-Thr, where X may be any amino-acid. Mannose in the cell is used to build up glycosyl groups.

For example, if the β-glucanase gene is isolated from a strain of Bacillus subtilis, the enzyme can be expressed in and secreted from yeast after hybridisation to the above-mentioned secretion signal, both at the DNA level. The enzyme contains a suitable trigger sequence, because it is glycosylated on secretion.

EP-A-0171000 discloses a method of producing a protein or peptide, which comprises culturing yeast transformant immobilised on a carrier gel, the transformant being transformed by a secretor expression vector. The secretion factor ensures that a desired polypeptide can be secreted from yeast. The polypeptide may contain one or more glycosylation signals, but the polypeptide which is produced may not be glycosylated if, as seems likely, any glycosylation signal is usually cleaved. For example, the photograph reproduced as Figure 11 does not suggest that IL-2 has been glycosylated.

WO-A-8603777 discloses that the pre-region of premelibiase can be used in engineering secretion, from genetically-modified yeast, or peptides and proteins other than melibiase.

EP-A-0201208 (published 12.11.86, i.e. after the priority date of 01.09.86 claimed in this application) discloses "supersecreting" mutants of Saccharomyces cerevisiae. It includes a definition of the word processing as "(a) the loss or proteolytic cleavage of the signal sequence from the polypeptide or protein so as to produce the polypeptide or protein in mature form; and/or (b) the addition of oligosaccharide to the glycosylation recognition sequences which are inherently present or may be provided by added clycosylation sequences in the mature protein". The mature protein is that actually secreted by the cell, by "processing" a precursor polypeptide or protein, as synthesised within a cell, having a signal sequence attached to the mature form. The purpose or practice of this oligosaccharide addition is unclear.

The gene for encoding bacterial β-lactamase occurs naturally and is found on many of the currently-used cloning vectors, as a selectable marker. The gene confers on the host resistance to ampicillin and other penicillin derivatives. When introduced into yeast by transformation, the gene is weakly expressed from its own promoter; it has been expressed at much higher levels by using various yeast promoters to drive the expression (Hollenberg, C.P. (1982) Current Topics Microbiol. Immunol., 96 119-144). In addition, secretion signals from other sources have been fused to the coding region of the mature protein to facilitate secretion in the host Saccharomyces cerevisiae (Mileham et al., unpublished observations; see also Leicester Biocentre Annual Report 1985; and Yeast (1986) Special Issue vol. 2).

Surprisingly, it has been found that, while many polypeptides (including β-glucanase as produced by its natural host Bacillus subtilis) are degraded by the proteolytic enzymes or are otherwise rendered unstable in a yeast culture supernatant, β-glucanase in its glycosylated form is stable in this medium.

### SUMMARY OF THE INVENTION

According to the present invention, a glycosylated analogue of a selected polypeptide containing no glycosylation signal is produced by expression and secretion from yeast in a fermentation process; to achieve this end, DNA encoding a glycosylation signal and (if necessary) a secretion signal is inserted into the organism which is then cultured. It is likely that the glycosylated analogue has the same activity and superior stability.

## DESCRIPTION OF THE INVENTION

The (non-glycosylated) selected polypeptide may be naturally secreted from the organism, i.e. a secretion signal (which is usually cleaved on secretion) is encoded and expressed. If it is not, the insertion must also encode the secretion signal.

Brewer's or distiller's yeast may be a natural host for expression of the selected polypeptide. However, the invention can also be applied if the polypeptide is foreign to the yeast; in this case, the insertion must also encode the polypeptide.

By the process of the present invention, a "selected" polypeptide encoded by yeast DNA is modified in up to three ways. Firstly, the structure of the expressed polypeptide includes a secretion signal, e.g. the signal region of α-factor when the host is to be S. cerevisiae. Secondly, the structure as expressed includes a glycosylation signal, e.g. an amino-acid triplet as given above. Thirdly, on secretion, it is glycosylated. All of these three factors (or as many of them as are required) can be organised so that the function of the selected polypeptide is effectively unchanged, while glycosylation provides stability.

The genetic information encoding the glycosylated polypeptide may be carried on a plasmid vector. A plasmid, e.g. a circular plasmid, for use in the invention, comprises a first sequence which allows for growth in a yeast in cell culture and a second sequence which codes for a polypeptide including both a eukaryotic secretion signal and a eukaryotic glycosylation signal. Such a plasmid may be cloned, and maintained in a suitable host, e.g. E. coli, as desired.

A plasmid for use in the invention may be especially constructed to take account of the stability which has been found for glycosylated polypeptides. Thus, the genetic information in the plasmid may be the same as that in known vectors which have been modified to encode a secretion factor and in which the glycosylation factor is part of the structure of the expressed polypeptide. Such polypeptides, e.g. the bacterial enzyme β-glucanase, are glycosylated naturally on secretion from the unnatural host S. cerevisiae.

Alternatively, the information encoding the glycosylated polypeptide is stably integrated into the yeast chromosome. Suitable techniques are described in EP-A-0231608.

The selected polypeptide may be any polypeptide having a desired function in the brewing process, e.g. a non-naturally-occurring protein such as a hybrid enzyme or other semi-synthetic protein, or a naturally-occurring enzyme. The gene for the polypeptide can be isolated by a conventional procedure, cloned (if desired), and inserted into a plasmid for use in the invention or otherwise into the DNA of the vector.

In order that the coding sequence should code for secretion (if necessary) and glycosylation signals, conventional site-directed mutagenesis may be used. Site-directed mutagenesis should minimise structural changes to the selected polypeptide. In certain circumstances, however, it may be preferable to use a procedure involving cloning of oligonucleotides containing the desired glycosylation site on to the gene either at the terminus or within the cloning region. This procedure is exemplified below.

Site-directed mutagenesis is a technology well-documented by Eckstein and co-workers (Taylor et al, Nucleic Acids Res. 13, 8749-8785). In this case, the point mutation required may be determined by the use of a synthetic oligonucleotide spanning the region to be mutated. This oligonucleotide is designed to have a mismatch as required. Shown beloware the region of interest in the mature β-lactamase gene, the mutated region in the β-lactamase product, and the corresponding oligonucleotide (which may be made synthetically) required in the procedure to generate the 'glycosylation' site:

5' GTG.AGA.ATA.GTG.TAT.GCG 3'
5' GTG.AGA.ATA.GTT.TAT.GCG 3'
3' CAC.TCT.TAT.CAA.ATA.CGC 5'

In the lowest strand, the glycosylation site Asn-Tyr-Ser is evident.

A host containing plasmid containing the β-lactamase region may be subjected to site-directed mutagenesis, using the synthetic oligonucleotide and reagents and procedures in a commerically-available kit (Amersham system code RPN 2322), Colonies may then be chosen and the vectors contained therein screened by DNA sequencing, e.g. using the dideoxy chain termination method. The complete β-lactamase gene is then constructed and the modified gene is introduced into yeast. For stability studies, it is necessary to incorporate on to the plasmid a yeast DNA origin of replication and a selectable marker.

It will usually be appropriate to insert the appropriate sequences in a terminal region of the expressed polypeptide, in order that its function should be substantially unchanged. Of course, if in fact that functional polypeptide is dependent on a terminal structure, another site or sites for insertion of the relevant base sequences can be chosen, either by experiment or on the basis of knowledge of the polypeptide's structure-activity relationship.

The invention is not limited to the objective of protection or stabilisation against protease-degraded polypeptides. As a general matter, it is possible to use recombinant techniques to produce protease-free yeast strains, but this is not practical for yeasts used in brewing.

The invention can be used for the production of a glycosylated analogue of, say, α-amylase, a foam-stabilising protein, β-amylase, pullulanase, amyloglucosidase or any other peptide of industrial significance or value, e.g. a pharmaceutically-active peptide.

The following Example illustrates the invention, using as a model bacterial β-lactamase. The Example illustrates cloning of an oligonucleotide containing the glycosylation site on to the gene, either at the terminus of the enzyme coding region or within the coding region itself.

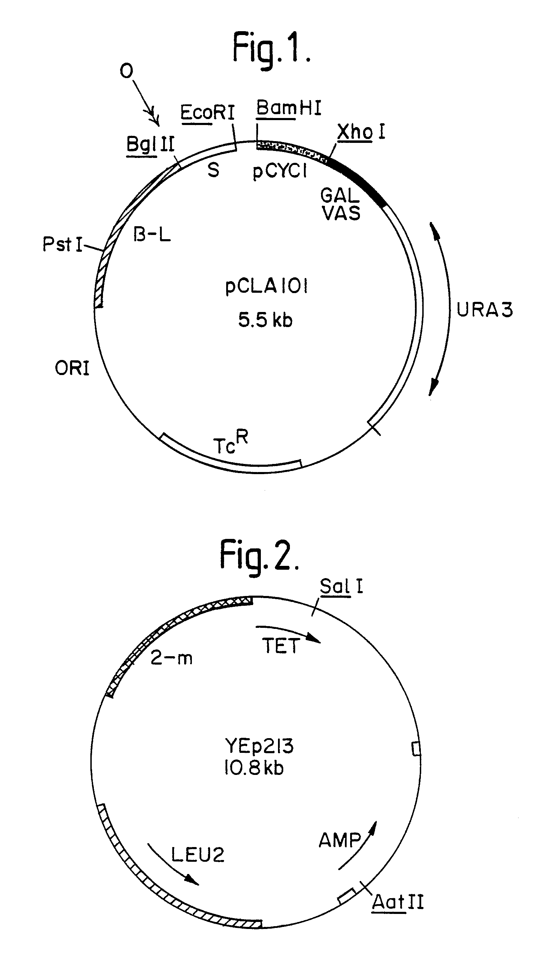

The abbreviations used in the accompanying Figures 1 to 3 have the following meanings:

→ indicates site of inclusion of oligonucleotide

β-L = β-lactamase

2-m = 2-micron

pCYC1 = promoter from yeast cytochrome C1 gene

GAL UAS = Upstream Activation Sequence from yeast gene GAL

Tc $^R$ = tegracycline resistance gene

OR1 = E. coli origin of replication

S̄ = secretion signal of β-lactamase

O = oligonucleotide

### Example

The starting plasmid was pCLA101 (see Figure 1; also disclosed by A. Mileham et al., in preparation). This bacterial plasmid contains the β-lactamase expression system. Expression of the β-lactamase is under the control of the yeast CYC1 gene promoter which in turn is regulated by the Upstream Activation Sequence taken from the yeast GAL1/GAL10 gene. The latter permits gene expression to be turned on by growth in galactose-containing medium. In this plasmid, the β-lactamase has been modified such that a recognition site for the restriction endonuclease BglII has been introduced to coincide with the point at which the secretion signal is cleaved to produce the mature enzyme. This site is used here to insert the glycosylation site-containing oligonucleotide.

Two single-stranded oligonucleotides were synthesised (using an Applied Biosystems 380B); they were annealed together by heating to 80°C, and allowed to cool to 4°C, to yield the following structure:-

GAT.CTG.AAC.GAA.ACT.AGC

AG.TTG.CTT.TGA.TCG.CTA.G

Asn Glu Thr Ser Asp

Clearly the sequence Asn-Glu-Thr is a potential glycosylation recognition site. It is seen that both 'sticky ends' are compatible with BglII-cleaved 'sticky ends' but only the end to the left as drawn would re-ligate to such an end to reform a BglII site. A sample (approx. 1 μg) of this mixture was ligated into vector pCLA101 which had been cleaved with restriction endonuclease BglII. After transformation into Escherichia colistrain 5K ( thi⁻, thr⁻1, leuB6, lacY1, tonA21, supE44 r$_k$⁻ m$_k$⁻), and selection of positive colonies on Lauria Broth (LB) medium containing 50 μg/ml tetracycline, clones were screened by the Grunstein & Hogness colony hybridisation technique (Proc. Natl. Acad. Sci. (1975) USA 72, 3961-3965) using the above oligonucleotide as a probe after kinase-labelling with ³²P-gamma-adenosine triphophate (Maniatis (1982) A Laboratory Manual CSH, page 123). Positive colonies identified after X-ray film exposure were then isolated and plasmid DNA prepared (Birnboim & Doly (1979) Nucleic Acids Res. 7, 1513).

The correct orientation of insertion of oligonucleotide into the BglII site is very important as the glycosylation signal must remain in frame with the β-lactamase enzyme coding region. The orientation of the insertion in the clones was determined by the dideoxy DNA sequencing method. Several examples of the clones were each cleaved with the two restriction enzymes EccRI and PstI, and the released fragment ligated into sequencing vector M13mp19 which had been similarly cleaved. One of the clones, named pCLA101-0, was identified as containing the oligonucleotide cloned in the required orientation.

## Conversion to yeast vector

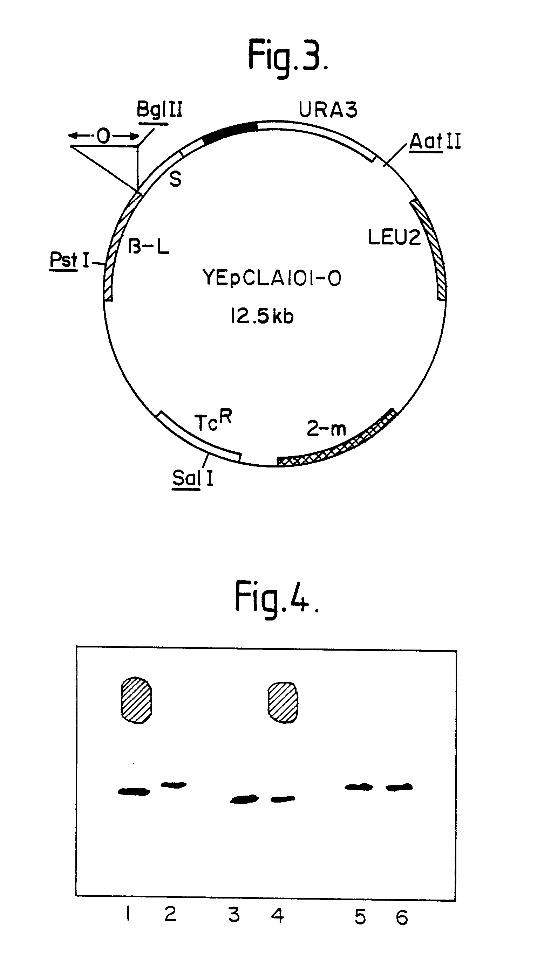

In order to be able to introduce the modified gene into yeast for stability studies, it was necessary to incorporate a yeast DNA origin of replication and a selectable marker. This was achieved using standard genetic construction techniques (see Maniatis (1982) A Laboratory Manual CSH). Plasmid YEp213 (see Fig. 2; Sherman et al, Methods in Yeast Genetics, Cold Spring Harbor, 1986) was used as a source of yeast replicon and selectable marker (LEU 2 gene). The large AatII-SalI fragment contains the required sequences and this was substituted for the Aat II-SalI fragment in pCLA101-0 by cleavage with SalI and AatII, and ligation. This product vector is called YEpCLA101-0 (see Fig. 3).

## Yeast transformation

The plasmid YEpCLA101-0 (approx. 1 μg) was transformed into yeast strain JRY188 (α leu2-3, 122 his3ΔI trpl-289 ura3-52 sir3-8) using the lithium salt mediated procedure (Ito et al . (1983) J. Bacteriol. 153, 163-168). Selection of plasmid containing transformants was made on synthetic medium lacking leucine.

## Cultures for β-lactamase studies

Two yeast strains were used for comparative studies. One was the transformant described above containing the newly constructed plasmid YEpCLA101-0 and the other was a control strain containing plasmid YEpCLA101. This latter plasmid is identical to YEpCLA101-0 except that the oligonucloeotide containing the glycosylation site is not present.

Strains were cultured in liquid galactose-containing medium (1% w/v yeast extract, 1% w/v bacteriological peptone, 2% w/v galactose) until Optical Density at 600 nm reached approximately 10. The culture fluids were cleared of cells by centrifuging at 10,000 X G for 10 minutes. The culture supernatants were concentrated 25 fold by ultrafiltration using Amicon Centriflow cones (CF25, molecular weight cut-off 25,000).

## Gel electrophoresis studies

Polyacrylamide slab electrophoresis (native) was performed in 10% gels (Maurer (1971), Disc Electrophoresis and Related Techniques of Polyacrylamide Gel Electrophoresis, de Gruyter, Berlin, 44-45). Activity of β-lactamase in the gel bands was determined according to a published procedure (Roggenkamp et al. (1981) Proc. Natl. Acad. Sci. USA 78, 4466-4470). The enzyme activity was visualised by staining using the penicillin analogue nitrocefin (5 mg/ml solution).

Some samples were pretreated with the enzyme endoglycosidase H to remove the glycosylation chains from the protein core. This incubation was performed overnight (Anal. Biochem. (1984) 141, 515-522).

Figure 4 is a diagrammatic representation of a typical native gel.

For the results depicted in Fig. 4, culture supernatants were concentrated 25-fold. The gel was loaded with 20 μl sample per track. This volume contained approximately 25 mg total protein of which approximately one-third is β-lactamase. In the case of the endoH-treated samples, half this amount of protein was present.

In Fig. 4, track 1 is for untreated culture A (YEpCLA101-0 in JRY188) and track 2 is for supernatant concentrate C (YEpCLA101 in JRY188). Tracks 3 and 4 are respectively for A+(endoH-treated culture supernatant concentrate) and A-(minus endoH, control culture supernatant concentrate). Tracks 5 and 6 are respectively for C+(endoH-treated culture supernatant concentrate) and C-(minus endoH, control culture supernatant control).

Comparison of tracks 1 and 2 shows that the introduction of the oligonucleotide has led to a considerable proportion of enzyme being glycosylated. The activity in the smear at the top of the gel corresponds to high molecular weight glycosylated protein. Comparison of tracks 3 and 4 shows that the high molecular weight smear is removed by the treatment, confirming it to be a glycosylated form of the enzyme.

## Stability studies

A primary purpose of this invention is to increase the stability of a foreign protein by deliberate introduction of glycosylation into the protein produced by yeast. One test relevant to brewing which determines protein stability is the protein's resistance to or tolerance of proteolytic enzymes. The effects of the enzyme trypsin on the above-mentioned enzyme preparations have been studied with a view to determining that stability in comparison to their non-glycosylated analogues.

30 $\mu$l of 100 mg/ml trypsin were added to 50 $\mu$l each of the concentrated yeast culture supernatants (approx. 0.1 mg/ml protein). Samples were incubated at 37 C for 15 minutes. Immediately after this, the samples were assayed for $\beta$-lactamase activity (Roggenkamp et al , ref. as above) by adding the whole of 820 $\mu$l of 0.1 M phosphate buffer pH 7.2. To this were added 50 $\mu$l of 0.5 mg/ml nitorcefin solution. The enzyme reaction course was followed by measuring change in absorbance at 490 nm against time.

The results, which are means of triplicate assays, are given in the following Table. They show that the glycosylated, modified enzyme is significantly more resistant to trypsin inactivation than the unmodified control. In the Table, activities are expressed in terms of arbitary spectrophotometer units per minute per $OD_{600}$ unit of original yeast culture. The figures are percentages of activity remaining after treatment compared to the activities in the untreated samples.

The results demonstrate the effectiveness of the process in stabilising foreign proteins against proteolytic attack.

6

| SAMPLE | ENZYME ACTIVITY | |
| --- | --- | --- |
| | UNITS | % OF UNTREATED |
| **A(YEpCLA101-0)** | | |
| Control Sample | 1260.0 | 100.0 |
| Test Samples: 1 | 92.7 | 7.3 |
| 2 | 87.3 | 6.9 |
| 3 | 80.2 | 6.3 |
| Mean | 86.73 | 6.83 |
| Standard Deviation | +/- 6.3 | +/- 0.5 |
| **C(YEpCLA101)** | | |
| Control Sample | 641.0 | 100.0 |
| Test Samples: 1 | 13.44 | 2.1 |
| 2 | 14.93 | 2.3 |
| 3 | 10.97 | 1.7 |
| Mean | 13.10 | 2.03 |
| Standard Deviation | +/- 2.0 | +/- 0.3 |

## Claims

1. A yeast fermentation process, e.g. in brewing or distillation, in which the yeast DNA has been modified such that a selected polypeptide not naturally secreted by the yeast in a glycosylated form is expressed and secreted in the form of a glycosylated analogue.

2. A process according to claim 1, in which the selected polypeptide is foreign to the yeast and the DNA is modified to encode also the polypeptide.

3. A process according to claim 1 or claim 2, in which the modification comprises site-directed mutagenesis.

4. A process according to claim 1 or claim 2, in which the modification comprises oligonucleotide cloning.

5. A process according to any preceding claim, in which the selected polypeptide is an enzyme having a function in the fermentation.

# Fig.1.

O

BglII    EcoRI    BamHI    XhoI

S    pCYCl

PstI    B-L    GAL VAS

pCLAlOl
5.5 kb

ORI    URA3

TcR

# Fig.2.

SalI

2-m    TET

YEp2l3
l0.8kb

LEU2    AMP

AatII

# Fig.3.

# Fig.4.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 185 327 (SUNTORY) * Page 10, lines 4-13; page 16, lines 3-25; page 20, lines 13-29 * | 1-3,5 | C 12 N 15/00 C 12 C 11/00 // C 12 N 9/86 |
| X | EP-A-0 126 206 (CETUS) * Page 12, lines 17-34; pages 30-36; pages 40-43; pages 45-47 * | 1-3,5 | |
| X | EP-A-0 171 000 (SUNTORY) * Page 2, line 28 - page 4, line 37; page 9, lines 24-27; page 10, lines 4-24; page 17, lines 28-31; page 25, line 27 - page 26, line 9; page 27, lines 7-19 * | 1-2,4 | |
| Y | CELL V13, 1978, pages 461-473; K. OLDEN et al.: "The role of carbonydrates in protein secretion and turnover: effects of tunicamycin on the major cell surface glycoprotein of chick embryo fibroblasts" * Abstract * | 1-5 | TECHNICAL FIELDS SEARCHED (Int. Cl.4) |
| Y | PROC. NATL. ACAD. SCI, vol. 81, September 1984, pages 5330-5334, US; G.A. BITTER et al.: "Secretion of foreign proteins from saccharomyces cerevisiae directed by alpha-factor gene fusions" * Page 5334, last paragraph * -/- | 1-5 | C 12 N C 12 C |

The present search report has been drawn up for all claims

| Place of search | Date of completion of the search | Examiner |
|---|---|---|
| THE HAGUE | 07-12-1987 | MADDOX A.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent
Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | PROCEEDINGS OF THE NATIONAL ACADEMY OF SCIENCES OF THE USA, vol. 81, no. 21, November 1984, pages 6594-6598, Washington, US; T. CABEZON et al.: "Expression of human alpha1-antitrypsin cDNA in the yeast saccharomyces cerevisiae" * Page 6595, right-hand column, last paragraph - page 6596, right-hand column; page 6596, right-hand column, last paragraph * | 1-5 | |
| A | BIOTECHNOL. GENET. ENG. REV., vol. 3, 1985, pages 377-416, Intercept Ltd, S.M. KINGSMAN et al.: "Heterologous gene expression in saccharomyces cerevisiae" * pages 398-405 * | 1-5 | |
| A | CHEMICAL ABSTRACTS, vol. 101, 1984, page 222, abstract no. 85798t, Columbus, Ohio, US; L. LEHLE et al.: "Primary structural requirements for N- and O-glycosylation of yeast mannoproteins", & BIOCHIM. BIOPHYS. ACTA 1984, 799(3), 246-51 * Abstract * | 1-5 | TECHNICAL FIELDS SEARCHED (Int. Cl.4) |
| A | EP-A-0 143 081 (CIBA-GEIGY) * Pages 7,8,86,87 * | 1-5 | |
| A | TRENDS IN BIOTECHNOLOGY, vol. 3, no. 2, 1985, pages 51-53, P.W. BERMAN et al.: "Engineering glycoproteins for use as pharmaceuticals" * Whole document * | 1-5 | |

-/-

The present search report has been drawn up for all claims

| Place of search | Date of completion of the search | Examiner |
|---|---|---|
| THE HAGUE | 07-12-1987 | MADDOX A.D. |

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | EP-A-0 201 208 (COLLABORATIVE RESEARCH) * Page 10, lines 10-17; page 13, lines 2-27 * | 1-4 | |

TECHNICAL FIELDS SEARCHED (Int. Cl.4)

The present search report has been drawn up for all claims

| Place of search | Date of completion of the search | Examiner |
|---|---|---|
| THE HAGUE | 07-12-1987 | MADDOX A.D. |